(19) Europäisches Patentamt
European Patent Office
Office européen des brevets

(11) Publication number: **0 439 186 A2**

(12) **EUROPEAN PATENT APPLICATION**

(21) Application number: **91100977.7**

(22) Date of filing: **25.01.91**

(51) Int. Cl.5: **C07C 309/14**, C07C 229/16, C07C 229/12, C07D 295/15, C07D 295/088, B01F 17/00, C11D 1/90, C11D 1/92

(30) Priority: **26.01.90 JP 16701/90**

(43) Date of publication of application: **31.07.91 Bulletin 91/31**

(84) Designated Contracting States: **DE ES FR**

(71) Applicant: **KAO CORPORATION**
**14-10, Nihonbashi Kayabacho 1-chome**
**Chuo-ku Tokyo(JP)**

(72) Inventor: **Konomi, Ohta**
**5-28, Nishi Takamatsu 1-chome**
**Wakayama-shi, Wakayama(JP)**
Inventor: **Hiroshi, Abe**
**1450, Nishihama**
**Wakayama-shi, Wakayama(JP)**
Inventor: **Jun, Aikawa**
**1130, Nishihama**
**Wakayama-shi, Wakayama(JP)**
Inventor: **Uichiro, Nishimoto**
**173, Otani**
**Wakayama-shi, Wakayama(JP)**
Inventor: **Kohshiro, Sotoya**
**446-26, Nakaguro Iwadecho**
**Naga-gun, Wakayama(JP)**

(74) Representative: **Hansen, Bernd, Dr. Dipl.-Chem. et al**
**Hoffmann, Eitle & Partner Patent- und Rechtsanwälte Arabellastrasse 4**
**W-8000 München 81(DE)**

(54) **Novel betaine and dispersant composition containing the same.**

(57) Novel betaines of formulas (1) and (2):

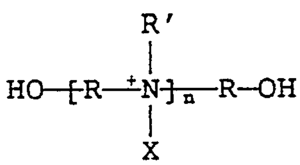

(1)

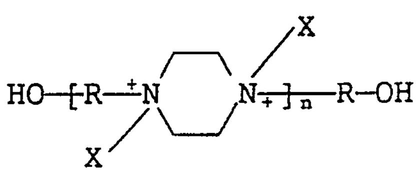

(2)

wherein R represents a straight or branched alkylene group containing from 2 to 24 carbon atoms, an alicyclic alkylene group, an aralkylene group or a group of the formula

EP 0 439 186 A2

$$-(CH_2CH_2O)_p-(CH_2CH_2S)_q-$$

(in which p represents 0 or a positive integer and q represents a positive integer), R' represents a straight or branched alkyl group containing from 1 to 24 carbon atoms or an aralkyl group, n represents a positive integer of from 2 to 50 and X represents group of the formula $-(CH_2)_mCOO^-$ (m being 1 or 3) or $-CH_2CH(OH)CH_2SO_3^-$; and dispersants containing the betaines are disclosed.

# NOVEL BETAINE AND DISPERSANT COMPOSITION CONTAINING THE SAME

## FIELD OF THE INVENTION

The present invention relates to novel betaines and dispersant compositions containing the same.

## BACKGROUND OF THE INVENTION

Polybetaines having cationic groups in their side chains are known and have been dealt with in a number of reports.

They can be readily produced by polymerizing a betaine-modified olefin. Thus, for example, West German Patent 2658118 describes a method of producing sulfobetaine-modified olefins and a method of producing copolymers of such olefins. There is a similar report on carbobetaines as well.

On the other hand, polybetaines having cationic groups in their principal chain can be produced by converting tertiary polyamines to betaines. However, for the production of the polyamines so far disclosed, multistep processes are required which start with long-chain alkylamines and involve repetitions of cyanoethylation, reaction with a haloalkylamine, and/or addition of ethylene oxide followed by amination. Therefore, there have been many restrictions concerning their structure.

## SUMMARY OF THE INVENTION

Accordingly, it is an object of the present invention to provide polybetaines having a novel structure starting with tertiary polyamines obtainable by a simple process.

Another object is to provide dispersant compositions containing the polybetaines.

The present inventors made intensive investigations in an attempt to overcome the drawbacks of the prior art such as mentioned above and, as a result, have now completed the present invention.

The present invention thus provides novel betaines of formula (1) or (2):

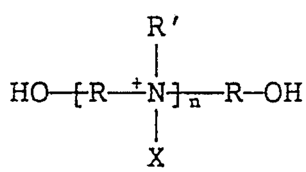

(1)

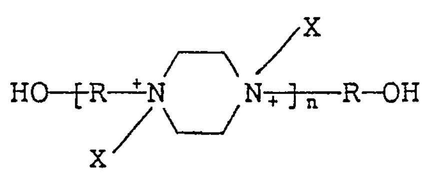

(2)

wherein R represents a straight or branched alkylene group containing from 2 to 24 carbon atoms, an alicyclic alkylene group, an aralkylene group or a group of the formula

$$-(CH_2CH_2O)_p-(CH_2CH_2)_q-$$

(in which p represents 0 or a positive integer and q represents a positive integer), R' represents a straight or branched alkyl group containing from 1 to 24 carbon atoms or an aralkyl group, n represents a positive integer of from 2 to 50 and X represents a group of the formula $-(CH_2)_mCOO^-$ (in which m represents 1 or 3) or $-CH_2CH(OH)CH_2SO_3^-$.

## BRIEF DESCRIPTION OF THE DRAWINGS

Fig. 1 is a graph showing the results of measurement for absorbance of dispersions in Test Example.

DETAILED DESCRIPTION OF THE INVENTION

In the above formulas (1) and (2), the alicyclic alkylene group represented by R contains preferably from 5 to 6 carbon atoms, the aralkylene group represented by R contains preferably from 6 to 10 carbon atoms and the aralkyl group represented by R' contains preferably from 18 to 22 carbon atoms.

In the above formulas (1) and (2), R' is preferably benzyl group or phenetyl group, and p is preferably 0 or a positive integer of from 1 to 3 and q is preferably a positive integer of from 1 to 10.

The betaines of formula (1) or (2) can be respectively produced by converting a tertiary amino alcohol of formula (3) or (4) shown below to a hydroxysulfobetaine or carbobetaine.

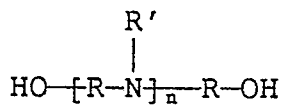

(3)

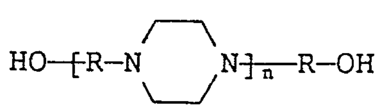

(4)

In formulas (3) and (4), R, R' and n are as defined above.

The tertiary amino alcohols of general formula (3) and of formula (4) are the subject matter of another invention made by the present inventors and disclosed in Japanese Patent Application No. Hei-1-219046 (corresponding to U.S. Patent Application Serial No. 07/563,712).

Among the novel betaines according to the present invention, those hydroxysulfobetaines in which, in formula (1) or (2), X is $-CH_2CH(OH)CH_2SO_3^-$ can be produced by the method as described, for example, in British Patent 1541427 or Swiss Patent 373047, namely by reacting epichlorohydrin with sodium bisulfite and then reacting the resulting 1-halo-2-hydroxypropane-3-sulfonic acid alkali metal salt with a tertiary amino alcohol of formula (3) or (4). Among the novel betaines according to the present invention, those carbobetaines in which, in formula (1) or (2), X is $-(CH_2)_mCOO^-$ (m being 1 or 3) can be produced by the method described, for example, in U.S. Patent 2,217,846, namely by reacting a tertiary amino alcohol of formula (3) or (4) with sodium chloroacetate for converting the tertiary amino alcohol to a betaine.

Structurally, these novel betaines of the present invention have two characteristic features. One is that they have a plurality of cationic groups in their principal chain. For that reason, they can exhibit a modified mode of adsorption and show some characteristic properties such as an improved dispersing or flocculating properties. Another feature is that they have, on each end thereof, a hydroxyl group, which is a convertible functional group. Therefore various substituents can be introduced thereto and the betains of the present invention can be applied in much more practices.

Generally, surfactants having a plurality of hydrophilic groups have low surface activity because of difficulty in their forming micelles but many of them have distinguishing features from the dispersing, flocculating and/or solubilizing properties viewpoint. It has been confirmed that the novel hydroxysulfobetaine and carbobetaine compounds according to the present invention are also superior in dispersing power as compared to polyacrylic acid sodium salt (molecular weight about 7,000) which has been used so far as an organic builder. Therefore, the novel betaines according to the present invention are suited for use as dispersants.

Since they are compatible with other surface active substances, the novel betaines according to the present invention can be used also as detergents or detergent components. Furthermore, the range of choice is wide for the kinds of the alkyl group in the novel betaines according to the present invention and therefore they may also be used in nonaqueous systems when a more hydrophobic alkyl group is selected.

In addition to their use as dispersants, detergents and detergent components, the novel betaines according to the present invention can be used also as emulsifiers, antistatic agents, agents for petroleum recovery or antifogging agents, for instance.

The following examples illustrate the present invention in further detail but are by no means limitative of the scope of the present invention.

SYNTHESIS EXAMPLE

Synthesis of tertiary amino alcohol to be used as the starting material:

The tertiary amino alcohols to be used as the starting materials for betaines according to the present invention was synthesized in the following manner.

A reaction vessel equipped with an inlet tube for hydrogen and gaseous amine, and a condenser and a separator for condensing and separating water produced during a reaction, excess amine and oily distillate was charged with 1,200 g of 1,6-hexanediol and 4 % by weight, based on the alcohol, of a copper-based catalyst. The reaction system atmosphere was replaced with nitrogen gas and then the temperature of reaction system was elevated while introducing hydrogen gas. After maintaining the system temperature at 180 °C for 1 hour, monomethylamine was introduced into the system over 9 hours. Then the catalyst was removed and the desired product was obtained in a quantitative yield.

According to VPO (vapor pressure osmometric measurement) data, the product had a molecular weight of 422 and $\bar{n}$ (the mean value of n) was 2.7.

The product showed the following chemical shift values (in ppm) in its $^{13}$C-NMR spectrum. In the measurement, $CDCl_3$ was used.

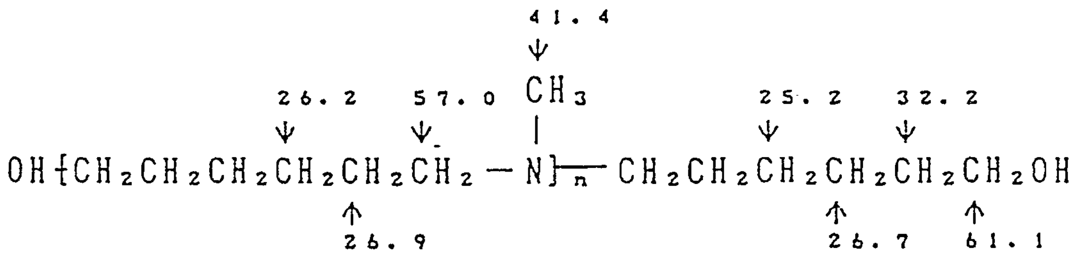


($\bar{n}$ = 2.7)

EXAMPLE 1

A four-necked flask equipped with a stirrer, a condenser, a dropping funnel and a thermometer was charged with 243.6 g of water, 76.3 g of sodium bisulfite and 0.279 g of NaOH and the mixture was heated to 60 °C. Then, 74.3 g of epichlorohydrin was added dropwise thereto. After completion of the dropping, the mixture was allowed to stand at 80 °C for 1 hour for maturation, followed by dropwise addition of 145.7 g of ethanol and 100 g of the tertiary amino alcohol synthesized in Synthesis Example. After completion of the dropping, maturation was performed at 90 °C. After the lapse of 1 hour following commencement of the maturation, 88.2 g of 5% aqueous Solution of NaOH was added dropwise to the reaction mixture over 5 hours followed by additional 4 hours of maturation.

The unreacted amine in the reaction mixture was quantitated by titration and the conversion rate was calculated based thereon. As the result, the conversion rate was 99.0%. The chloride ion, water and volatile matter contents were also determined and the composition of the reaction mixture at the time of termination of the reaction was estimated as follows:

| | |
|---|---|
| Sulfobetaine according to the present invention | 24.7% |
| Water | 43.0% |
| Ethanol | 20.6% |
| Sodium chloride | 6.2% |
| Unknown substances | 5.5% |

The sulfobetaine was isolated by evaporating the reaction mixture to dryness and adding an organic solvent for removing neutral salts. The product shows the following $^{13}C$-NMR spectrum:

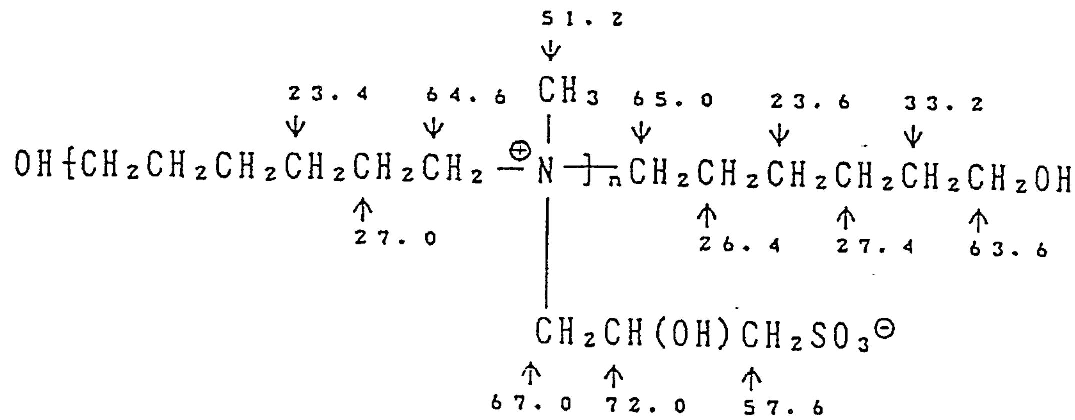

$(\bar{n} = 2.7)$

EXAMPLE 2

A four-necked flask equipped with a stirrer, a condenser, a dropping funnel and a thermometer was charged with a solution of 100 g of the tertiary amino alcohol synthesized in the synthesis example in 107.6 g of ethanol and the mixture was heated to 80° C. A solution of 93.6 g of sodium chloroacetate in 140.4 g of water was then added dropwise thereto. After completion of the dropping, the reaction mixture was maintained at 90° C for maturation. After the lapse of 1 hour following commencement of the maturation, a solution of 7.0 g of NaOH in 89.4 g of water was added dropwise to the reaction mixture over 5 hours, followed by additional 4 hours of maturation.

The unreacted amine in the reaction mixture was determined by titration and the conversion rate was calculated based thereon. As the result, the conversion rate was 95.8%. The chloride ion, water and volatile matter contents were also determined and the composition of the reaction mixture at the time of termination of the reaction was estimated as follows:

| | |
|---|---|
| Carbobetaine according to the present invention | 19.6% |
| Water | 42.7% |
| Ethanol | 19.6% |
| Sodium chloride | 8.2% |
| Unknown substances | 9.9% |

A $^{13}C$-NMR spectrum measured in the same manner as in Example 1 are shown below.

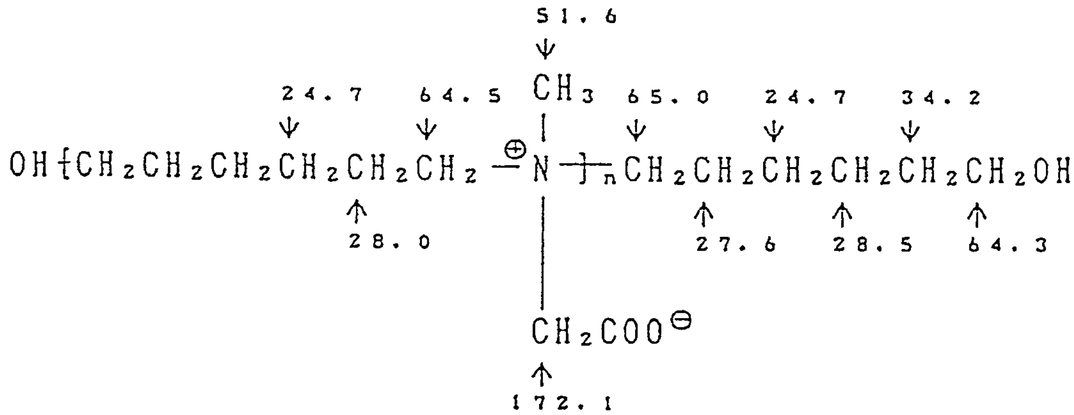

$(\overline{n} = 2.7)$

EXAMPLE 3

An amine of formula (3) in which R is

$$-(CH_2)_9-,$$

R' is $-CH_3$, MW is 750 and $\overline{n}$ is 3.8 was used and the reaction was carried out under the same conditions as in Example 1 or 2. A sulfobetaine and a carbobetaine were obtained at conversion rates of 86% and 88%, respectively.

EXAMPLE 4

An amine of formula (3) in which R is

$$-(CH_2CH_2O)_2-CH_2CH_2-,$$

R' is $-CH_3$, MW is 562 and $\overline{n}$ is 2.8 was used and the reaction was carried out under the same conditions as in Example 1 or 2. A sulfobetaine and a carbobetaine were obtained at conversion rates of 97% and of 90%, respectively.

EXAMPLE 5

An amine of formula (3) in which R is

$$-(CH_2)_6-,$$

R' is $-CH_2CH_2CH_2CH_3$, MW is 1530 and $\overline{n}$ is 9.1 was used and the reaction was carried out under the same conditions as in Example 1 or 2. A sulfobetaine and a carbobetaine were obtained at conversion rates of 92% and 94%, respectively.

EXAMPLE 6

An amine of formula (3) in which R

$-(CH_2)_6-$,

R' is $-CH_2C_6H_5$, MW is 666 and $\bar{n}$ is 2.9 was used and the reaction was carried out under the same conditions as in Example 1 or 2. A sulfobetaine and a carbobetaine were obtained at conversion rates of 95% and 92%, respectively.

EXAMPLE 7

An amine of formula (4) in which R is

$-(CH_2)_6-$,

MW is 650 and $\bar{n}$ is 2.3 was used and the reaction was carried out under the same conditions as in Example 1 or 2. A sulfobetaine and a carbobetaine were obtained at conversion rates of 88% and 88%, respectively.

TEST EXAMPLE

The betaines obtained in Examples 1-7 as described above and three dispersants for comparison, namely polyacrylic acid sodium salt (MW = 7,000), a hydroxysulfobetaine of formula (5) shown below and a carbobetaine of formula (6) shown below, were comparatively tested for their dispersing power by the test method mentioned below.

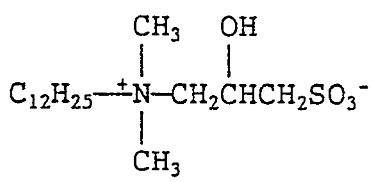

(5)

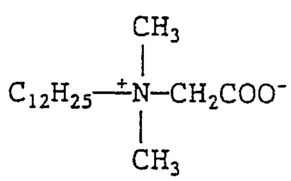

(6)

Test method:

50 ml of a 0.05% by weight aqueous solution of a sample, 0.05 g of clay or 0.01 g of carbon black were placed in a sedimentation tube. After vigorous shaking by hand, the mixture was treated with ultrasonic waves for 5 minutes and then allowed to stand for 2 hours in the case of clay or for 12 hours in the case of carbon black, followed by absorbance measurement at 420 nm. A pH of the water used was 8 and a hardness thereof was 0° DH, and the measurement temperature was 25° C.

The results thus obtained are shown graphically in Fig. 1.

A higher absorbance value indicates that the dispersing power for clay or carbon black is higher.

In Fig. 1, the results for the blank are the results obtained by adding clay or carbon black to the model water, shaking the mixture and allowing to stand under the same conditions as above and performing the absorbance measurement.

The results shown in Fig. 1 indicate that the novel betaines according to the present invention, when evaluated as dispersants, show high dispersing power.

While the invention has been described in detail and with reference to specific examples thereof, it will

be apparent to one skilled in the art that various changes and modifications can be made therein without departing from the spirit and scope thereof.

**Claims**

1. A betaine of formula (1) or (2):

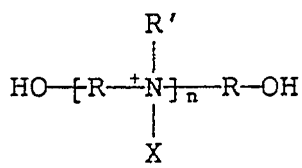

(1)

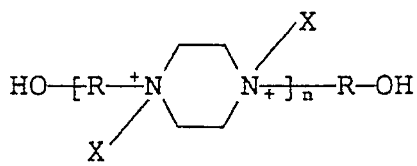

(2)

wherein R represents a straight or branched alkylene group containing from 2 to 24 carbon atoms, an alicyclic alkylene group, an aralkylene group or a group of the formula

$$-(CH_2CH_2O)_p-(CH_2CH_2)_q-$$

(in which p represents 0 or a positive integer and q represents a positive integer), R' represents a straight or branched alkyl group containing from 1 to 24 carbon atoms or an aralkyl group, n represents a positive integer of from 2 to 50 and X represents a group of the formula $-(CH_2)_mCOO^-$ (in which m represents 1 or 3) or $-CH_2CH(OH)CH_2SO_3^-$.

2. A betaine as claimed in Claim 1, wherein R' is a benzyl or phenethyl group.

3. A betaine as claimed in Claim 1 or 2, wherein R is

$$-(CH_2CH_2O)_p-(CH_2CH_2)_q-,$$

p represents 0 or an integer of from 1 to 3 and q represents an integer of from 1 to 10.

4. A dispersant composition which comprises the betaine of Claim 1, 2 or 3.

Fig. 1

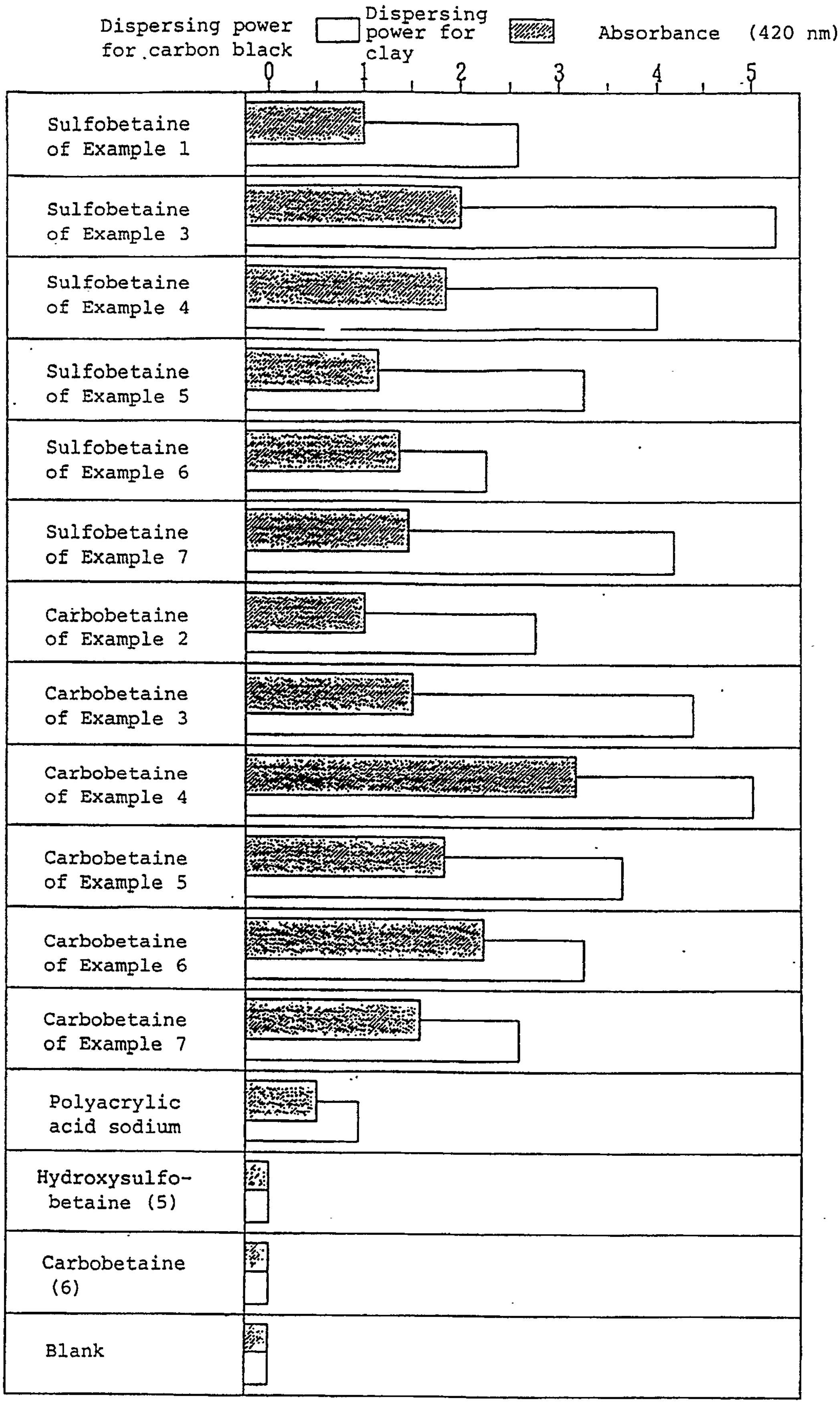
Dispersing power for carbon black
Dispersing power for clay
Absorbance (420 nm)
0
1
2
3
4
5
Sulfobetaine of Example 1
Sulfobetaine of Example 3
Sulfobetaine of Example 4
Sulfobetaine of Example 5
Sulfobetaine of Example 6
Sulfobetaine of Example 7
Carbobetaine of Example 2
Carbobetaine of Example 3
Carbobetaine of Example 4
Carbobetaine of Example 5
Carbobetaine of Example 6
Carbobetaine of Example 7
Polyacrylic acid sodium
Hydroxysulfo-betaine (5)
Carbobetaine (6)
Blank